# EUROPEAN PATENT SPECIFICATION

(11) **EP 2 839 738 B1**
(45) Date of publication and mention of the grant of the patent: **14.06.2017**
(21) Application number: 13778760.2
(22) Date of filing: 19.04.2013
(51) Int. Cl.: A01K 67/033

(54) **METHOD FOR BREEDING AND TRANSPORTING PREDATORY ARTHROPODS USING A SHELTER**
METHODE ZUM ZÜCHTEN UND TRANSPORTIEREN RÄUBERISCHER ARTHROPODEN MIT EINEM SCHUTZRAUM
PROCÉDÉ D'ÉLEVAGE AINSI QUE DE TRANSPORT DE ARTHROPODE PRÉDATEURS METTANT EN OUVRE UN ABRI

(30) Priority: 20.04.2012 JP 2012097134
(43) Date of publication of application: 25.02.2015
(73) Proprietor: Ishihara Sangyo Kaisha, Ltd., Osaka-shi, Osaka 550-0002 (JP)
(72) Inventor: YOSHIDA, Kiyomitsu, Kusatsu-shi Shiga 525-0025 (JP); HIRANO, Kohji, Kusatsu-shi Shiga 525-0025 (JP); MORI, Kotaro, Kusatsu-shi Shiga 525-0025 (JP); OHASA, Makiko, Kusatsu-shi Shiga 525-0025 (JP)
(74) Representative: Müller-Boré & Partner Patentanwälte PartG mbB
(86) International application number: PCT/JP2013/061670
(87) International publication number: WO 2013/157640

(56) References cited:
- EP-A1- 0 777 963
- JP-A- H09 154 436
- JP-A- H11 253 069
- JP-A- 2002 047 116

## Description

The present invention relates to a method for rearing and a method for transporting predatory arthropods using a shelter, wherein the natural enemies are predatory arthropods, particularly natural enemies that are predatory insects..

Utilization of natural enemies that are predatory insects to control harmful insects that damage crops requires mass rearing of the predatory insects. Generally, since the number of individual predatory insects often decreases due to cannibalization during rearing, a method for rearing natural enemies by using paper, chaff, buckwheat chaff, or the like as a shelter has been recommended. Further, a method for rearing natural enemies using a foamed plastics shelter is disclosed in Patent Literature 1.

Patent Literature 1
Japanese Patent Application Publication, Tokukaihei, No. 9-154436 A (Publication Date: June 17, 1997)

Since, in the case of rearing or in the case of transporting natural enemies efficiently in large quantities in a container, it is difficult to sufficiently prevent cannibalization with use of a conventional shelter, there has been a demand for a method that improves the effect of preventing cannibalization during rearing or transportation.

The present invention is intended to solve the aforementioned problems and provides a method for rearing and transporting natural enemies, the method including using a shelter including a group of sheet compositions containing cellulose fibers.

The present invention makes it possible to prevent predatory arthropods from cannibalizing one another in a container and rear or transport them efficiently in large quantities. Further, use of the shelter makes it possible to release natural enemies easily and efficiently by, in releasing the natural enemies, placing or disposing in a desired place the group of sheet compositions containing cellulose fibers or a sheet composition separated from the group. The group of sheet compositions or the sheet composition thus placed or disposed have preferable environmental advantages because they can be easily disposed of by a method such as incineration, together with plants that are to be discarded after crop harvest or the like, and, what is more, because they are biodegradable.
Fig. 1 is a perspective view showing the appearance of a shelter used according to an embodiment of the method of the present invention.
Fig. 2 is a perspective view showing the appearance of a container used according to an embodiment of the method of the present invention.

An embodiment of the present invention is described in detail below with reference to the drawings. For convenience of explanation, members having the same functions are given the same reference signs and are not repeatedly described.

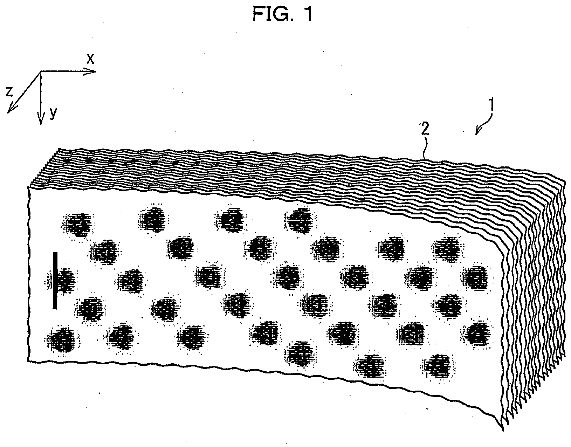

Fig. 1 is a perspective view showing the appearance of a shelter 1 used according to an embodiment of the present invention. In the drawing, the x axis, the y axis, and the z axis define directions in a three-dimensional space in the drawing, respectively. As used herein, the "horizontal" length of the shelter refers to the length of the shelter along the x axis in Fig. 1, and the "vertical" length of the shelter refers to the length of the shelter along the y axis in Fig. 1. Further, as used herein, the thickness of a sheet composition or a bundle of sheet compositions (i.e. the shelter) refers to the length of the shelter along the z axis in Fig. 1.

The shelter 1 is a shelter that is used in the rearing or transportation of natural enemies. The shelter 1 is a bundle of sheet compositions 2. Each of the sheet compositions 2 contains cellulose fibers and has an uneven surface. In other words, the shelter 1 is a group of sheet compositions 2. This configuration allows a natural enemy, i.e. a target of rearing, to hide itself in a gap between one sheet composition and another and/or in a gap between cellulose fibers constituting the sheet compositions. This in turn reduces for example the chance of natural enemies meeting one another, thus making it possible to reduce the possibility of cannibalization.

In a shelter used according to the method of the present invention, each of the sheet compositions bundled together serves as a barrier to movement of natural enemies. In other words, a natural enemy hiding in a gap between one sheet composition and another is isolated from another natural enemy by the sheet compositions. Further, since, in a gap formed between one sheet composition and another, the sheet compositions touch the body of a natural enemy, the gap provides a suitable environment for the natural enemy, which is attracted by a narrow space. Therefore, the shelter used according to the method of the present invention can more efficiently reduce the chance of natural enemies meeting one another than a shelter made of an unbundled single piece of paper or a shelter made of foamed plastic.

As used herein, the term "sheet composition" is intended to mean a product formed in the shape of thin paper. The thickness of each of the sheet compositions is for example, but is not limited to, 50 to 300 µm.

An example of a material for the sheet composition containing cellulose fibers is, but is not limited to, at least one material selected from the group consisting of paper, virgin pulp, recycled pulp, and cellulose nonwoven fabric. Among them, paper and/or virgin pulp are/is desirable.

Examples of paper serving as the material for cellulose fibers include tissue paper, paper made from virgin pulp such as kitchen towels (also called "paper towels" or "kitchen rolls") and animal paper flooring materials, newspaper, copy paper, Japanese paper, simili paper, cardboard, coarse paper, toilet paper, etc. Among them, paper having a moisture-retaining property and an uneven surface is preferable, and paper made from virgin pulp is specifically preferable.

An example of virgin pulp serving as the material for cellulose fibers is pulp made directly from wood material or non-wood material and used mainly for making paper. Examples of wood pulp include an N material derived from a conifer such as a fir tree or a pine tree and an L material derived from a broad-leaved tree such as a eucalyptus or a poplar. Examples of non-wood pulp include straw - pulp, bagasse pulp, reed pulp, kenaf pulp, mulberry pulp, etc. Among them, virgin pulp having a moisture-retaining property and many voids and virgin pulp having an uneven surface are preferable.

An example of recycled pulp serving as the material for cellulose fibers is pulp made from recycled paper such as used paper or offcut and used for making paper. Further, an example of cellulose nonwoven fabric is a sheet of cloth made by joining fibers derived from cellulose such as wood pulp with an adhesive or an adhesive force of fibers per se. Among them, recycled pulp having a moisture-retaining property and many voids and recycled pulp having an uneven surface are preferable.

With a moisture-retaining property, the sheet compositions can favorably provide a humidity that attracts natural enemies. Further, with many voids and with an uneven surface, the sheet compositions favorably make it easier for natural enemies to hide. By saying that the sheet compositions "have voids", it is meant that there is a gap between cellulose fibers constituting the sheet compositions and there are small voids in surfaces of the sheet compositions.

The number of sheet compositions to be bundled together can be appropriately adjusted according to the type of paper serving as the material, the type and number of natural enemies to be reared or transported, the type and size of the container used, etc., and as such, cannot be unconditionally determined. However, the number ranges preferably from about 2 to 100, more preferably from about 10 to 50, even more preferably from about 15 to 30. Depending on the various conditions, however, the number does not need to fall within any of these ranges.

Further, according to the present invention, the sheet compositions thus bundled together are fixed or pressure-bonded at at least one given part of each of the sheet compositions. This configuration favorably makes it possible that a gap between one sheet composition and another that allows a natural enemy, i.e. a target of rearing or transportation, to hide itself can be maintained throughout the stages from rearing to transportation. Appropriately usable examples of means for fixing include, but are not particularly limited to, a stapler, a string, a clip, etc.

As used herein "pressure-bonded" means the state of sheet compositions bundled together without use of the fixing means such as a stapler, a string, or a clip. For example, in the case where the material for the sheet compositions is virgin pulp or recycle pulp, the sheet compositions may be bundled together in a pressure-bonded state without use of the fixing means such as a stapler, a string, or a clip. The "pressure-bonded" state is intended, for example, to mean the state of a plurality of sheet compositions brought into intimate contact at their given parts by pressure generated by cutting the sheet compositions put on top of each other.

The parts at which the sheet compositions are fixed or pressure-bonded may be, but are not particularly limited to, ends of the sheet compositions or centers of the sheet compositions.

The shape of the shelter used according to the method of the present invention is not particularly limited as long as it fits the purpose of the present invention. However, for example, it is preferable to use a shelter obtained by fixing a plurality of strip sheet compositions at one end. It is preferable that each of the strip sheet compositions have dimensions, for example, of about 1 to 10 cm × about 1 to 10 cm. It is preferable that a bundle of these strip sheet compositions have a thickness, for example, of about 0.5 to 10 cm, more preferably about 0.5 to 3 cm. Predatory arthropods that are reared or transported by the present invention are predatory arthropods such as insects, spiders, and mites.

Examples of the insects include: predatory *Thysanoptera* such as *Haplothrips brevitubus*, *Franklinothrips vespiformis*, and *Haplothrips chinensis*; predatory *Anthocoridae* such as *Orius strigicollis* and *Orius sauteri*; *Chrysopidae* such as *Chrysoperla carnea*, *Chrysoperla nipponensis*, and *Chrysopa pallens*; *Coccinellidae* such as *Harmonia axyridis*, *Coccinella septempunctata,* and *Propylaea japonica*; *Harpalidae* such as *Chlaenius pallipes* and *Chlaenius micans*; and *Dermaptera* such as *Labidura riparia*; and the like.

Examples of the spiders include: *Salticidae* such as *Hasarius adansoni*, *Plexippus paykulli*, and *Menemerus brachygnathus*; *Lycosidae* such as *Pardosa astrigera*, *Pardosa pseudoannulata*, and *Pirata subpiraticus*; and the like.

Examples of the mites include: *Phytoseiidae* such as *Phytoseiulus persimilis*, *Neoseiulus californicus*, *Neoseiulus cucumeris*, *Amblyseius swirskii*, *Neoseiulus womersleyi*, *Amblydromalus limonicus*, *Gynaeseius liturivorus*, *Neoseiulus barkeri*, and *Iphiseius degenerans*; and the like.

A rearing method of the present invention is achieved by placing at least one bundle of sheet compositions as a shelter in a rearing place in rearing natural enemies according to the usual rearing method (e.g. see T. Yushima et al. ed., "Rearing methods of insects" issued by Japan Plant Protection Association). The size of the shelter to be used varies depending on the type and number of natural enemies. However, it is preferable that the shelter be placed or packed to occupy about 5 to 100% by volume (including voids between materials) of the rearing container. Depending on the various conditions, however, the size does not need to fall within this range.

A specific example of operation is a method including (a) releasing (disposing) 2 to 1400, preferably 500 to 900, eggs of predatory arthropods serving as natural enemies, e.g. predatory insects, per 250 ml of a rearing container and (ii) feeding them as appropriate. Feed can be selected as appropriate according to the type and number of natural enemies to be reared. Examples of feed include: pollen; animal protein; eggs, larvae, and adults of arthropods; and the like. As an example, in a case where the predatory arthropods are *Haplothrips brevitubus*, eggs of *Lepidoptera* insects or the like can be used.

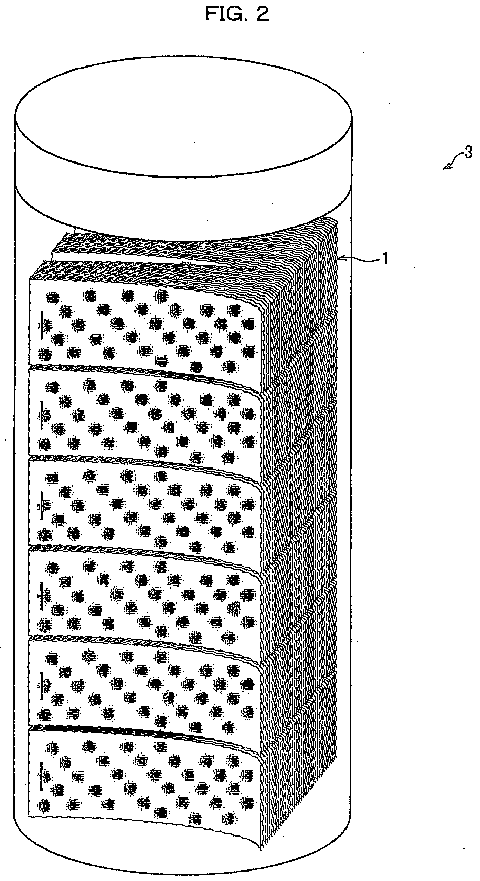

A transporting method of the present invention may be achieved, for example, by (a) placing or packing a shelter according to the present invention in a transporting container of predatory arthropods serving as natural enemies and (b) charging the predatory arthropods and, as needed, their feed. A method for placing or packing a paper-bundled shelter or a method for disposing or using natural enemies and feed is the same as in the case of the aforementioned rearing method of natural enemies. Further, it is possible to directly use the rearing container at the time of transportation. Fig. 2 is a perspective view showing the appearance of a container 3 according to an embodiment of the method of the present invention. In the container 3, shelters 1 are placed or packed.

Next, some preferred embodiments of the method of the present invention are exemplified. Note, however, that these embodiments are not intended to limit the present invention.
<1> A method for rearing or transporting natural enemies, the method comprising the step of: using a shelter comprising a group of sheet compositions containing cellulose fibers and having an uneven surface, wherein the shelter is a bundle of the sheet compositions, and the sheet compositions thus bundled together are fixed or pressure-bonded at at least one given part of each of the sheet compositions.
<2> The method as set forth in <1>, wherein the shelter is a bundle of 2 to 100 of these sheet compositions.
<3> The method as set forth in <1>, wherein the shelter is shaped such that the sheet compositions are fixed at one end, the sheet compositions being each in the shape of a strip.
<4> The method as set forth in <3>, wherein each of the strip sheet compositions has dimensions of 1 to 10 cm × 1 to 10 cm and a bundle of the strip sheet compositions has a thickness or 0.5 to 10 cm.
<5> The method as set forth in <1>, wherein each of the sheet compositions is at least one selected from the group consisting of paper, virgin pulp, recycled pulp, and cellulose nonwoven fabric.
<6> The method as set forth in <1>, wherein each of the sheet compositions is at least either one of paper and virgin pulp.
<7> The method as set forth in <1>, wherein each of the sheet compositions is paper.
<8> The method as set forth in <1>, wherein a container in which the shelter is placed or packed in a proportion of 5 to 100% (including voids between materials) by volume of the container is used.

### [Examples]

For a more detailed description of the present invention, Examples are described below. Note, however, that the present invention should not be interpreted within the limits of these Examples. The animal paper flooring materials used in the present Examples are bundles of strip animal paper flooring materials.

### <Example 1>

Twenty strips each measuring 3 cm × 7 cm were prepared from a kitchen towel (manufactured by Oji Nepia Co., Ltd.; paper made from virgin pulp). A single paper-bundled shelter (about 10% by volume, including voids) was prepared by fixing the twenty strips (measuring about 1 cm thick) at one end with a stapler, and was put into a polystyrene cup having a volume of 250 ml. Furthermore, a piece of water-saturated cotton (measuring 2 cm in diameter × 2 cm × 0.5 cm) was put in a plastic petri dish, and the petri dish was placed in the container. Seven hundred eggs of *Haplothrips brevitubus* were released into the polystyrene cup, and were fed with *Ephestia kuehniella* eggs. The feed was replenished as appropriate during rearing. After fifteen days, the survival rate was obtained by counting the number of insects that had survived. The test was repeated six or ten times, and the average survival rate is shown in the first table. For comparison, the first table also shows the average survival rates obtained in a case where corrugated filter paper (measuring 9 cm in diameter) was used as a shelter, a case where polystyrene foam measuring 5 mm in diameter was used as a shelter, a case where vermiculite was used as a shelter, and a case where no shelter was used, respectively.

**[Table 1]**

| First Table | | | | | | |
|---|---|---|---|---|---|---|
| | Shelter | Number of eggs introduced | Repeat count | Average survival rate (%) | Standard deviation (SD) | Coefficient of variation (CV) |
| Present invention | Paper bundle | 700 | 10 | 78.8 | 6.0 | 0.08 |
| Comparison group | Corrugated filter paper | 700 | 10 | 59.0 | 7.0 | 0.12 |
| | Polystyrene foam | 700 | 10 | 63.7 | 3.4 | 0.05 |
| | Vermiculite | 700 | 6 | 52.2 | 16.1 | 0.31 |
| | None | 700 | 6 | 60.2 | 3.2 | 0.05 |

As a result of evaluation according to Tukey's test (p<0.05), it was statistically confirmed that there are significant differences between the average survival rate of the present invention and the average survival rates of the comparison group.

### <Example 2>

Twenty strips each measuring 3 cm × 7 cm were prepared from a kitchen towel (manufactured by Oji Nepia Co., Ltd.). A single paper-bundled shelter (about 10% by volume, including voids) was prepared by fixing the twenty strips (measuring about 1 cm thick) at one end with a stapler, and was put into a polystyrene cup having a volume of 250 ml. Alternatively, 20 ml (about 10% by volume, including voids) of a strip animal paper flooring material (marketed as "Gokigen Kaiteki Matto" (manufactured by GEX Co., Ltd.)) measuring 1 cm × 2 cm was put into a polystyrene cup having a volume of 250 ml. Furthermore, a piece of water-saturated cotton (measuring 2 cm in diameter × 2 cm × 0.5 cm) was put in a plastic petri dish, and the petri dish was placed in the container. A hundred eggs of *Haplothrips brevitubus* were released into the polystyrene cup, and were fed with *Ephestia kuehniella* eggs. The feed was replenished as appropriate during rearing. After twenty-three days, the survival rate was obtained by counting the number of insects that had survived. The test was repeated six times, and the average survival rate is shown in the second table. For comparison; the second table also shows the average survival rate obtained in a case where vermiculite was used as a shelter.

**[Table 2]**

| Second Table | | | | | | |
|---|---|---|---|---|---|---|
| | Shelter | Number of eggs introduced | Repeat count | Average survival rate (%) | Standard deviation (SD) | Coefficient of variation (CV) |
| Present invention | Paper bundle | 100 | 6 | 93.2 | 3.3 | 0.04 |
| | Animal paper flooring material | 100 | 6 | 88.0 | 5.4 | 0.06 |
| Comparison group | Vermiculite | 100 | 6 | 58.3 | 9.9 | 0.17 |

As a result of evaluation according to Tukey's test (p<0.05), it was statistically confirmed that there are significant differences between the average survival rates of the present invention and the average survival rate of the comparison group.

### <Example 3>

Three paper-bundled shelters (about 25% by volume, including voids) were each prepared by fixing twenty strips (measuring about 1 cm thick) at one end with a stapler, and were put into a polystyrene cup having a volume of 250 ml. The strips, each measuring 3 cm × 7 cm, had been prepared from a kitchen towel (manufactured by Oji Nepia Co., Ltd.). Four first instar larvae, four second instar larvae, and four third instar larvae (twelve in total) of *Chrysoperla nipponensis* were released into the polystyrene cup, and were fed with *Ephestia kuehniella* eggs. The feed was replenished as appropriate during rearing. After twelve days, the survival rate was obtained by counting the number of insects that had survived. The test was repeated three times, and the average survival rate is shown in the third table. For comparison, the third table also shows the average survival rate obtained in a case where no shelter was used.

**[Table 3]**

| Third Table | | | | | | |
|---|---|---|---|---|---|---|
| | Shelter | Number of larvae introduced | Repeat count | Average survival rate (%) | Standard deviation (SD) | Coefficient of variation (CV) |
| Present invention | Paper bundle | 12 | 3 | 79.6 | 15.1 | 0.19 |
| Comparison group | None | 12 | 3 | 43.5 | 21.2 | 0.49 |

As a result of evaluation according to t-test (p<0.01), it was statistically confirmed that there is a significant difference between the average survival rate of the present invention and the average survival rate of the comparison group.

### <Example 4>

Three paper-bundled shelters (about 25% by volume, including voids) were each prepared by fixing twenty strips (measuring about 1 cm thick) at one end with a stapler, and were put into a polystyrene cup having a volume of 250 ml. The strips, each measuring 3 cm × 7 cm, had been prepared from a kitchen towel (manufactured by Oji Nepia Co., Ltd.). Four first instar nymphs, four third instar nymphs, and four fifth instar nymphs of *Orius strigicollis* were released into the polystyrene cup, and were fed with *Ephestia kuehniella* eggs. The feed was replenished as appropriate during rearing. After ten days, the survival rate was obtained by counting the number of insects that had survived. The test was repeated three times, and the average survival rate is shown in the fourth table. For comparison, the fourth table also shows the average survival rate obtained in a case where no shelter was used.

**[Table 4]**

| Fourth Table | | | | | | |
|---|---|---|---|---|---|---|
| | Shelter | Number of nymphs introduced | Repeat count | Average survival rate (%) | Standard deviation (SD) | Coefficient of variation (CV) |
| Present invention | Paper bundle | 12 | 3 | 41.7 | 8.3 | 0.20 |
| Comparison group | None | 12 | 3 | 13.9 | 4.8 | 0.35 |

As a result of evaluation according to t-test (p<0.01), it was statistically confirmed that there is a significant difference between the average survival rate of the present invention and the average survival rate of the comparison group.

### <Example 5>

Five paper-bundled shelters (about 20% by volume, including voids) were each prepared by fixing twenty strips (measuring about 1 cm thick) at one end with a stapler, and were put into a polyethylene bottle having a volume of 500 ml. The strips, each measuring 3 cm × 7 cm, had been prepared from a kitchen towel (manufactured by Oji Nepia Co., Ltd.). Alternatively, 100 ml (about 10% by volume, including voids) of a strip animal paper flooring material (marketed as "Gokigen Kaiteki Matto" (manufactured by GEX Co., Ltd.)) measuring 1 cm × 2 cm was put into a polyethylene bottle having a volume of 500 ml. A hundred imagoes of *Haplothrips brevitubus* were released into the polyethylene bottle. After the insects had been left at normal temperature for one day in the similitude of transportation, the survival rate was obtained by counting the number of insects that had survived. The test was repeated four times, and the average survival rate is shown in the fifth table. For comparison, the fifth table also shows the average survival rate obtained in a case where vermiculite was used as a shelter.

**[Table 5]**

| Fifth Table | | | | | | |
|---|---|---|---|---|---|---|
| | Shelter | Number of imagoes introduced | Repeat count | Average survival rate (%) | Standard deviation (SD) | Coefficient of variation (CV) |
| Present invention | Paper bundle | 100 | 4 | 86.3 | 11.5 | 0.13 |
| | Animal paper flooring material | 100 | 4 | 88.8 | 14.0 | 0.16 |
| Comparison group | Vermiculite | 100 | 4 | 47.5 | 18.5 | 0.39 |

As a result of evaluation according to Tukey's test (p<0.05), it was statistically confirmed that there are significant differences between the average survival rates of the present invention and the average survival rate of the comparison group.

A rearing method, and a transporting method of the present invention make it possible efficiently rear and transport natural enemies.

### Reference Signs List

- 1: Shelter
- 2: Sheet composition
- 3: Container

## Claims

1. A method for rearing or transporting predatory arthropods, the method comprising the step of:
using a shelter comprising a group of sheet compositions containing cellulose fibers and having an uneven surface, wherein:
the shelter is a bundle of the sheet compositions; and
the sheet compositions thus bundled together are fixed or pressure-bonded at at least one given part of each of the sheet compositions.

2. The method as set forth in claim 1, wherein the shelter is a bundle of 2 to 100 of these sheet compositions.

3. The method as set forth in claim 1, wherein the shelter is shaped such that the sheet compositions are fixed at one end, the sheet compositions being each in the shape of a strip.

4. The method as set forth in claim 3, wherein each of the strip sheet compositions has dimensions of 1 to 10 cm by 1 to 10 cm and a bundle of the strip sheet compositions has a thickness of 0.5 to 10 cm.

5. The method as set forth in claim 1, wherein each of the sheet compositions is at least one selected from the group consisting of paper, virgin pulp, recycled pulp, and cellulose nonwoven fabric.

6. The method as set forth in claim 1, wherein each of the sheet compositions is paper.

7. The method as set forth in claim 1, wherein each of the sheet compositions is paper made from virgin pulp.

8. The method as set forth in claim 1, wherein:
a container in which the shelter is placed or packed in a proportion of 5 to 100% (including voids between materials) by volume of the container is used.

## Patentansprüche

1. Verfahren zum Aufziehen oder Transportieren von räuberischen Gliederfüßlern, wobei das Verfahren den Schritt umfaßt:
Verwenden eines Unterschlupfs, umfassend eine Gruppe von blattförmigen Zusammensetzungen, die Cellulosefasern enthalten und eine unebene Oberfläche aufweisen, wobei:
der Unterschlupf ein Bündel der blattförmigen Zusammensetzungen ist und
die so zusammengebündelten blattförmigen Zusammensetzungen an mindestens einem vorgegebenen Teil von jeder der blattförmigen Zusammensetzungen fixiert oder unter Druck verpresst sind.

2. Verfahren wie in Anspruch 1 dargestellt, wobei der Unterschlupf ein Bündel von 2 bis 100 dieser blattförmigen Zusammensetzungen ist.

3. Verfahren wie in Anspruch 1 dargestellt, wobei der Unterschlupf so geformt ist, daß die blattförmigen Zusammensetzungen an einem Ende fixiert sind, wobei die blattförmigen Zusammensetzungen jeweils in Form eines Streifens sind.

4. Verfahren wie in Anspruch 3 dargestellt, wobei jede der streifenförmigen blattförmigen Zusammensetzungen Dimensionen von 1 bis 10 cm mal 1 bis 10 cm aufweist und ein Bündel der streifenförmigen blattförmigen Zusammensetzungen eine Dicke von 0,5 bis 10 cm aufweist.

5. Verfahren wie in Anspruch 1 dargestellt, wobei jede der blattförmigen Zusammensetzungen mindestens eine, ausgewählt aus der Gruppe, bestehend aus Papier, nativer Pulpe, wiederaufbereiteter Pulpe und nicht verwebter Cellulosestruktur ist.

6. Verfahren wie in Anspruch 1 dargestellt, wobei jede der blattförmigen Zusammensetzungen Papier ist.

7. Verfahren wie in Anspruch 1 dargestellt, wobei jede der blattförmigen Zusammensetzungen Papier ist, das aus nativer Pulpe hergestellt ist.

8. Verfahren wie in Anspruch 1 dargestellt, wobei:
ein Behälter, in dem der Unterschlupf zu einem Anteil von 5 bis 100% (einschließlich Leerräumen zwischen den Materialien), bezogen auf das Volumen des Behälters, plaziert oder verpackt ist, verwendet wird.

## Revendications

1. Procédé d'élevage ou de transport d'arthropodes prédateurs, le procédé comprenant l'étape suivante:
utiliser un abri comprenant un groupe de compositions de feuilles comprenant des fibres de cellulose et présentant une surface irrégulière, dans lequel:
l'abri est constitué d'un ensemble de compositions de feuilles; et
les compositions de feuilles ainsi mises ensemble sont fixées ou liées par pression sur au moins une partie donnée de chacune des compositions de feuilles.

2. Le procédé selon la revendication 1, dans lequel l'abri est un ensemble de 2 à 100 de ces compositions de feuilles.

3. Le procédé selon la revendication 1, dans lequel l'abri est mis en forme de telle sorte que les compositions de feuilles sont fixées à une extrémité, les compositions de feuilles étant chacune sous la forme d'une bande.

4. Le procédé selon la revendication 3, dans lequel chacune des compositions de feuilles en bande présentant des dimensions de 1 à 10 cm sur 1 à 10 cm et un ensemble des compositions de feuilles en bande présente une épaisseur de 0,5 à 10 cm.

5. Le procédé selon la revendication 1, dans lequel chacune des compositions de feuilles est au moins l'une sélectionnée parmi le groupe composé de papier, de pâte vierge, de pâte recyclée et de tissu non tissé de cellulose.

6. Le procédé selon la revendication 1, dans lequel chacune des compositions de feuilles est du papier.

7. Le procédé selon la revendication 1, dans lequel chacune des compositions de feuilles est du papier fabriqué à partir de pâte vierge.

8. Le procédé selon la revendication 1, dans lequel:
un conteneur dans lequel l'abri est placé ou emballé dans une proportion de 5 à 100% en volume du conteneur (en ce compris les vides entre les matières) est utilisé.
